# EUROPEAN PATENT APPLICATION

(11) **EP 4 700 070 A1**
(43) Date of publication of application: **25.02.2026**
(21) Application number: 24792514.2
(22) Date of filing: 04.04.2024
(51) Int. Cl.: C08H 7/00, A61K 8/97, A61Q 1/12, A61Q 17/04, C08L 97/00, C09D 11/08, C09D 197/00

(54) **KRAFT LIGNIN AND METHOD FOR PRODUCING SAME**

(30) Priority: 18.04.2023 JP 2023067712
(71) Applicant: DIC Corporation, Tokyo 174-8520 (JP)
(72) Inventor: SHOMURA, Kenji, Kamisu-shi, Ibaraki 314-0193 (JP)
(74) Representative: TBK
(86) International application number: PCT/JP2024/013870
(87) International publication number: WO 2024/219239

(57) **Abstract**

A problem of the present invention is to provide a cosmetic, an ink, a printed matter, a paint, an automotive paint, a coating, a plastic, a fiber, a film, a black matrix resist, a photo spacer resist and a cured material thereof containing a kraft lignin which cuts absorption in the ultraviolet region and which has absorption ability in the ultraviolet region and the like. It was found that, because a kraft lignin having specific peaks in an infrared absorption spectrum measured with a Fourier transform infrared spectrophotometer having an ATR unit has high ultraviolet-cutting ability and has a high L* value in color measurement with a spectrophotometer, the kraft lignin can be preferably used especially for cosmetic uses, and the problem could be thus solved.

## Description

### TECHNICAL FIELD

The present invention relates to a kraft lignin and a production method thereof.

### BACKGROUND ART

Ultraviolet rays are generally said to accelerate skin aging and to be causes of wrinkles and sagging or causes of blotches and skin cancer. Ultraviolet rays of the so-called UV-B region of 280 to 320 nm affect the surface layer of the skin, and thus excessive exposure is apt to cause blotches or skin cancer, while ultraviolet rays of the so-called UV-A region of 320 to 380 nm penetrate into the skin, and thus excessive exposure is apt to cause wrinkles and sagging.

For the purpose of protecting the skin from ultraviolet rays, sunscreens using an ultraviolet-cutting agent such as an ultraviolet absorber and an ultraviolet-scattering agent are widely used in the daily lives. In recent years, however, in view of protecting the environment and reducing harm to health of the human body, a trend to ban the use of such ultraviolet protectors, restrict them or consider them as a cause for concern has been spreading.

For example, Hawaii in the U.S. has passed a bill banning the sale of sunscreens that contain specific synthetic organic chemicals which are considered to be harmful to the coral reefs. Moreover, in Europe, the health hazard classification of titanium oxide has been raised to section 2 of the GHS classification under the REACH regulation. Furthermore, the toxicities of ultraviolet-scattering agents which are generally used for sunscreen compositions, such as zinc oxide and cerium oxide, on specific target organs are also section 1 or section 2, and the ultraviolet scattering-agents are not necessarily safe for the human body.

From such a background, natural product-derived ultraviolet absorbers are attracting attention. Although natural product-derived ultraviolet absorbers have been used for cosmetics including sunscreens in view of the safety, there is a problem because the dispersibility in an oil-based component is poor and because the cosmetics are washed off with sweat during the use since many thereof are water-soluble substances. For example, ultraviolet absorbers extracted from marine algae are derived from natural products and thus are sufficiently safe, but the ultraviolet absorbers are water soluble and have a problem of water resistance. Moreover, because the absorption edge is around 350 nm, the ultraviolet-protecting ability for UV-A is not sufficient compared to the ultraviolet-protecting ability for UV-B (PTLs 1 and 2). Ultraviolet absorbers derived from microbes inhabiting plants are similarly derived from natural products and are thus sufficiently safe, but the ultraviolet absorbers have a problem because the ultraviolet-absorbing ability for UV-B is not sufficient while the ultraviolet absorbers have ultraviolet-absorbing ability for UV-A. Moreover, there is also a problem of water resistance (PTL 3).

Here, an ultraviolet-cutting agent obtained by coating the surface of a pigment with an ultraviolet-cutting agent made of a naturally derived raw material, for improving the dispersibility in an oil-based component, in fact has improved dispersibility, but the inherent ultraviolet-absorbing ability is merely exerted (PTL 4).

Moreover, although there is a related art in which a naturally derived lignin is used as an additive for a lead storage battery, the lignin itself has dark brown to black appearance and thus has been used neither for cosmetic uses for the purpose of adding beautiful appearance nor for paint uses. (PTL 5)

For using for the cosmetic uses, the paint uses or the like, the lignin is preferably in the form of fine particles, but because the concentration for particle formation is very low and because the yield of lignin relative to the capacity of the production facility is low, there is a problem of very low productivity. (NPLs 1 and 2)

### CITATION LIST

### PATENT LITERATURE

PTL 1: JPH08-188521A
PTL 2: JP2004-238519A
PTL 3: JP2013-127027A
PTL4: JP2018-162400A
PTL 5: JP2003-331908A

### NON PATENT LITERATURE

NPL 1: Green synthesis of lignin nano- and micro-particles: Physicochemical characterization, bioactive properties and cytotoxicity assessment, International Journal of Biological Macromolecules 163 (2020) 1798-1809

NPL 2: Understanding Lignin Aggregation Processes. A Case Study: Budesonide Entrapment and Stimuli Controlled Release from Lignin Nanoparticles, ACS Sustainable Chem. Eng. 2018, 6, 9342-9351

### SUMMARY OF INVENTION

### TECHNICAL PROBLEM

A problem to be solved by the invention is to provide a cosmetic, an ink, a printed matter, a paint, an automotive paint, a coating, a plastic, a fiber, a film, a black matrix resist, a photo spacer resist and a cured material thereof containing a kraft lignin which cuts light in the ultraviolet region and which has absorption ability in the ultraviolet region and the like.

### SOLUTION TO PROBLEM

As a result of extensive research to solve the problem, the present inventor and the like have found that, because a kraft lignin having specific peaks in an infrared absorption spectrum measured with a Fourier transform infrared spectrophotometer using the attenuated total reflectance method (ATR method), which is one type of infrared spectroscopy, namely having an ATR unit (ATR-FT-IR below), has a high L* value in color measurement with a spectrophotometer and has high ultraviolet-absorbing ability, the kraft lignin can be preferably used especially for cosmetic uses, and the problem could be thus solved.

That is, the invention includes the following subject matters.

[1] A kraft lignin in which the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹) is in the range of 3.0 to 6.0 in an infrared absorption spectrum measured by ATR-FT-IR and in which the L* value is in the range of 61 to 80 in color measurement with a spectrophotometer.
[2] The kraft lignin according to 1 which has a median diameter D50 of 0.1 to 0.8 µm.
[3] The kraft lignin according to 1 or 2 in which the SPF (Sun Protection Factor) value measured using an SPF (Sun Protection Factor) analyzer is in the range of 10 to 50 when the coating amount of the kraft lignin per unit area is 1.0 mg/cm² in the measurement method of ISO24443.
[4] The kraft lignin according to any one of 1 to 3 in which the PA (Protection Grade of UVA) value measured using an SPF (Sun Protection Factor) analyzer is in the range of 10 to 50 when the coating amount of the kraft lignin per unit area is 1.0 mg/cm² in the measurement method of ISO24443.
[5] The kraft lignin according to any one of 1 to 4 in which the 2,2-diphenyl-1-picrylhydrazyl (DPPH) active radical-scavenging ability of the kraft lignin per unit weight is 1.0 to 3.0 mg DPPH/mg.
[6] A method for producing the kraft lignin according to any one of 1 to 5 characterized by having
   a step of dissolving a kraft lignin in an organic solvent and thus obtaining a kraft lignin solution,
   a subsequent step of adding ice or ice water to the kraft lignin solution or adding the kraft lignin solution to ice or ice water to deposit the kraft lignin and obtaining the deposited kraft lignin, and
   a subsequent step of filtering, washing and drying the deposited kraft lignin and thus obtaining the kraft lignin.
[7] A method for producing the kraft lignin according to any one of 1 to 5 characterized by having
   a step of dissolving a kraft lignin in an organic solvent and thus obtaining a kraft lignin solution,
   a subsequent step of adding an aqueous water-soluble metal salt solution to the kraft lignin solution or adding the kraft lignin solution to an aqueous water-soluble metal salt solution to deposit the kraft lignin, and
   a subsequent step of filtering, washing and drying the deposited kraft lignin and thus obtaining the kraft lignin.
[8] A cosmetic, an ink, a printed matter, a paint, an automotive paint, a coating, a plastic, a fiber, a film, a black matrix resist, a photo spacer resist and a cured material thereof containing the kraft lignin according to any one of 1 to 5 are provided.

### ADVANTAGEOUS EFFECTS OF INVENTION

The kraft lignin of the invention has a high SPF (Sun Protection Factor) value, a high PA (Protection Grade of UVA) value and high ultraviolet-scattering and reflecting ability. Moreover, because the L* value is high in color measurement with a spectrophotometer, the kraft lignin can be preferably used for a white-colored cosmetic, especially a sunscreen, a foundation, a face pack and a skin care cream. In addition, because the 2,2-diphenyl-1-picrylhydrazyl (DPPH) active radical-scavenging ability is high, the kraft lignin is effective for anti-aging, anti-wrinkle and anti-blotch when used as a cosmetic. Moreover, because the ultraviolet-absorbing ability is high, the kraft lignin can be used as a color material or an additive in a wide range of fields such as an ink, a printed matter, a paint, an automotive paint, a coating, a plastic, a fiber, a film, a black matrix resist, a photo spacer resist and a cured material thereof in addition to a cosmetic.

### BRIEF DESCRIPTION OF DRAWINGS

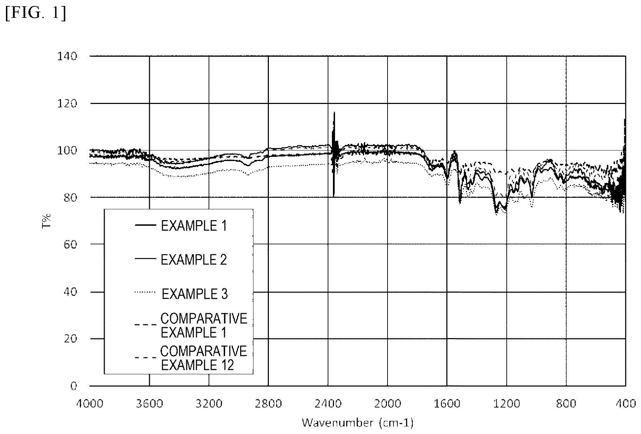

[FIG. 1] A figure showing the infrared absorption spectra of the kraft lignins of the invention measured by ATR-FT-IR.

### DESCRIPTION OF EMBODIMENTS

The embodiments of the invention shown below merely show some embodiments of the invention, and the invention is not limited to the described matters only as long as it does not depart significantly from the gist thereof.

### [Lignin]

Lignins are main components of plants, are phenolic polymer compounds highly contained in conifers, broad-leaved trees and gramineous plants, which are roughly classified, and play a role of adhering fibers in the plant cell walls of plants. Lignins are obtained from black liquor, which is a by-product obtained when pulp is produced by cooking wood chips, in the papermaking process.

### [Kraft Lignin]

Kraft lignin is one type of lignin. Depending on the cooking process, the chemical structure of the obtained lignin differs. Accordingly, the solubility in a solvent, including water, also largely differs. The invention is an invention related to a kraft lignin. Kraft lignin is a lignin obtained from black liquor, which generates as a by-product when pulp is obtained from wood chips by the kraft cooking process, and unlike lignosulfonate, which is a lignin obtained by the existing sulfite cooking process, kraft lignin is not easily dissolved in water, has a low molecular weight, contains many thiol groups, contains less sulfonic groups and has other differences.

The kraft lignin of the invention is a kraft lignin characterized in that the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹) is in the range of 3.0 to 6.0 in an infrared absorption spectrum measured by ATR-FT-IR and shows the characteristic infrared absorption spectrum, unlike known kraft lignins, because the kraft lignin has many hydroxy groups on the surface. The measurement conditions of ATR-FT-IR are shown below.

The measurement of FT-IR spectroscopy was conducted using a Fourier transform infrared spectrophotometer FT/IR-6100 manufactured by JASCO Corporation having an ATR unit.

Moreover, the kraft lignin of the invention is characterized by having a high amount of hydroxy groups, which are involved in antioxidative ability, and the hydroxy group amount was determined from the ratio of the heights of the peak of 3000 to 3600 cm⁻¹ showing the hydroxy groups and the peak of 1480 to 1540 cm⁻¹ showing the hydrocarbon groups which were measured by ATR-FT-IR.

The hydroxy group amount is in the range of 2.0 to 5.0 in terms of the above ratio of the heights, and the antioxidative ability is higher as the hydroxy group amount is higher. This is because the antioxidative ability depends on the amount of phenolic hydroxy groups, which capture free radicals.

Because only the part in contact with the prism is reflected to the measurement results in ATR-FT-IR, a kraft lignin in the form of finer particles has a higher ratio of the heights of the peak of 3000 to 3600 cm⁻¹ showing the hydroxy groups and the peak of 1480 to 1540 cm⁻¹ showing the hydrocarbon groups.

Moreover, the kraft lignin of the invention is characterized by having a median diameter D50 of 0.1 to 0.8 µm, and when the median diameter D50 is in the range of 0.1 to 0.8 µm, the kraft lignin of the invention is characterized by having a hue of white color and having a color with an L* value in the range of 61 to 80 in color measurement, while general kraft lignins have a hue of brownish red. Furthermore, the kraft lignin of the invention preferably has D50 of 0.15 to 0.6 µm. The L* value is preferably 65 to 80.

Moreover, the SPF value measured using an SPF analyzer was in the range of 10 to 50 when the coating amount of the kraft lignin per unit area was 1 mg/cm² in the measurement method of ISO24443, and it was found that the ultraviolet-cutting ability is far higher than those of known kraft lignins.

Furthermore, it was also found that the kraft lignin of the invention has high active radical-scavenging ability, namely 2,2-diphenyl-1-picrylhydrazyl (DPPH) active radical-scavenging ability per unit weight of the kraft lignin of 1.0 to 3.0 mg DPPH/mg.

Accordingly, because the kraft lignin of the invention has a hue of white color and has high ultraviolet-cutting ability, the kraft lignin can be preferably used for a sunscreen, a foundation, a face pack and a skin care cream. In addition, because the 2,2-diphenyl-1-picrylhydrazyl (DPPH) active radical-scavenging ability is high, the kraft lignin is effective for anti-aging, anti-wrinkle and anti-blotch when used as a cosmetic. Moreover, because the ultraviolet-absorbing ability is high, the kraft lignin can be used as a color material or an additive in a wide range of fields such as an ink, a printed matter, a paint, an automotive paint, a coating, a plastic, a fiber, a film, a black matrix resist, a photo spacer resist and a cured material thereof in addition to a cosmetic.

### [Production Method of Kraft Lignin]

The kraft lignin of the invention can be obtained by dissolving a kraft lignin in a good solvent such as an organic solvent and adding ice, ice water or an aqueous water-soluble metal salt solution to the kraft lignin solution or by dropping or adding the kraft lignin solution to ice, ice water or an aqueous water-soluble metal salt solution.

The kraft lignin of the invention is obtained by once dissolving a raw material kraft lignin in an organic solvent and then re-precipitating the kraft lignin. In general reprecipitation, in which a poor solvent which mixes in a polymer solution is added to deposit the polymer, the deposited polymer is precipitated in a size of 1 mm to several centimeters. The reason for precipitation in such a large size is that the polymer deposits in the state of adhesive fine particles and autoagglutinates during deposition.

In the invention, to prevent the autoagglutination and to obtain a submicron-size kraft lignin, ice, ice water or an aqueous water-soluble metal salt solution is used.

Specifically, as the poor solvent, ice, ice water or an aqueous water-soluble metal salt solution is added. When ice is added, because the kraft lignin is deposited in a state of being cooled with ice, the adhesiveness of the deposited fine particles is lost, and the good solvent is lost without agglutination of the deposited fine particles. Thus, the kraft lignin of the invention having the size of the deposited fine particles can be obtained. When ice water is added, for the same reason as that of the case of adding ice, the kraft lignin of the invention in the state of the deposited fine particles can be obtained. The ice water used in the invention is obtained by adding 20 parts by weight or more of ice to 100 parts by weight of water and refers to ice water at a temperature of 4°C or lower.

When an aqueous water-soluble metal salt solution is added as the poor solvent, the dissolved salt enters among the adhesive fine particles and prevents agglutination of the deposited fine particles, and the kraft lignin in the state of the deposited fine particles can be obtained.

Examples of the organic solvent which can be used for producing the kraft lignin of the invention include methanol, ethanol, ethylene glycol, diethylene glycol, triethylene glycol, polyethylene glycol, propylene glycol, dipropylene glycol, tripropylene glycol, polypropylene glycol, glycerin, butyl Cellosolve, butyl Carbitol, acetone, tetrahydrofuran (THF), dimethylformamide (DMF), dimethyl sulfoxide (DMSO) and N-methyl-2-pyrrolidinone (NMP), and especially, ethanol and ethylene glycol are preferably used. The organic solvent used is two to 20 times the raw material kraft lignin in terms of the weight ratio, and use of a small amount of the solvent is preferable also in view of the cost as long as the kraft lignin is dissolved. However, when the cost and the viscosity of the solution are considered, the organic solvent used is desirably three to 10 times.

Examples of the water-soluble metal salt which can be used for the aqueous water-soluble metal salt solution used for producing the kraft lignin of the invention include sodium chloride, potassium chloride, calcium chloride, magnesium chloride, sodium sulfate, potassium sulfate, calcium sulfate, magnesium sulfate, sodium acetate, potassium acetate, calcium acetate, magnesium acetate, sodium phosphate, potassium phosphate, calcium phosphate, magnesium phosphate, sodium nitrate, potassium nitrate, calcium nitrate, magnesium nitrate and the like, but a mixture salt of a plurality thereof may be used. Considering the cost, sodium chloride is most preferable.

Moreover, the aqueous water-soluble metal salt solution can be used at a concentration which results in a saturated aqueous solution in terms of the weight ratio or lower but is preferably used at a concentration of 1 to 20%.

The dissolution temperature of the organic solvent during the production of the kraft lignin of the invention largely affects the active radical-scavenging ability of the produced kraft lignin, and thus dissolution at 100°C or lower is preferable. More preferably, to maintain the active radical-scavenging ability, dissolution is conducted more preferably at room temperature. Similarly, the influence on the particle size is large, the temperature during the deposition is preferably controlled at 10°C or lower. Accordingly, the kraft lignin of the invention is deposited by adding ice to the kraft lignin solution or dropping or adding the kraft lignin solution to ice in an opposite manner. The particles of the kraft lignin of the invention are prepared without any heating step, which leads to coloring, and thus the kraft lignin has a hue of white color. Moreover, the production process does not require any difficult continuous process and is characterized by being able to prepare kraft lignin particles easily by a batch process.

Furthermore, although the substance to be dropped or added to deposit particles is a poor solvent to deposit the kraft lignin, with a water-alone-based system, the adhesive material immediately after deposition agglutinates, and large brown particles are formed. Accordingly, when water is used as the poor solvent, particles having a small particle size can be produced using a mixed solvent of water and a dispersant or an additive or of water and an organic solvent. As the deposition conditions, a system other than that above can also be used as long as the system can decrease the solubility of the kraft lignin and make the kraft lignin in a depositable state.

As the poor solvent, ice and cold water can be used, and especially, because the viscosity of the particles immediately after deposition can be easily decreased, ice can be preferably used. When ice, ice water or cold water is used, an additional equipment, such as an ice maker and a refrigerating machine, is required. Thus, considering the production cost, the aqueous water-soluble metal salt solution described below is preferably used.

For the aqueous water-soluble metal salt solution, sodium chloride, potassium chloride, calcium chloride, magnesium chloride, sodium sulfate, potassium sulfate, calcium sulfate, magnesium sulfate, sodium acetate, potassium acetate, calcium acetate, magnesium acetate, sodium phosphate, potassium phosphate, calcium phosphate, magnesium phosphate, sodium nitrate, potassium nitrate, calcium nitrate, magnesium nitrate and the like can be used, and of the aqueous water-soluble metal salt solutions thereof, sodium chloride is most preferable in view of the cost.

Moreover, the aqueous water-soluble metal salt solution can be used at a concentration which results in a saturated aqueous solution in terms of the weight ratio or lower but is preferably used at a concentration of 1 to 20%.

When the kraft lignin of the invention is used, the kraft lignin can be used in a wide range of fields such as an ink, a printed matter, a paint, an automotive paint, a coating, a plastic, a fiber, a film, a black matrix resist, a photo spacer resist and a cured material thereof in addition to a cosmetic. The uses described in detail below are examples, and the kraft lignin of the invention can be used as an additive for any use for the purpose of obtaining effects of protecting against ultraviolet rays, anti-oxidation, coloring and the like.

### (Cosmetic Use)

When the kraft lignin of the invention is used for a foundation, a cream or a sunscreen composition as an antioxidative agent or an ultraviolet protector, a clay mineral such as talc, synthetic phlogopite, mica and kaolin is used as an extender. As a synthetic inorganic powder, titanium oxide, boron nitride, zinc oxide, aluminum hydroxide, zinc chloride, lithium silicate, magnesium silicate, sodium silicate, tin oxide, iron oxide, barium sulfate, silica, hydrated silica or the like is used.

To add lubricity, a silicone polymer or fine polymer particles are used. As the silicone polymer, dimethicone, a (stearoxymethicone/dimethicone) copolymer, carboxydecyl trisiloxane, hydrogen dimethicone, tetrahydro tetramethylcyclotetrasiloxane, aminopropyl triethoxysilane, cyclomethicone, dimethiconol, trifluoroalkyldimethyl trimethylsiloxysilicate, hydrogen dimethicone or methicone is used. For the fine polymer particles, nylon-12, a (methyl methacrylate/acrylonitrile) copolymer, methyl methacrylate, a (hydrogenated polybutadiene/glycol/HDI) copolymer is used.

As an antioxidant or a preservative, BHT, phenoxyethanol, tocopherol, chlorphenesin, methylparaben, ethylparaben or the like is used.

As a moisturizer, sodium acetylated hyaluronate, sodium hyaluronate, water-soluble collagen or the like is used.

As an organic material which forms an organic layer, glyceryl tri(caprylate/caprate), stearic acid, dextrin palmitate, ethylhexylglycerin, glycerin, isobutane, isopentane, tetracene, lauroyl lysine, ascorbic acid, olive fruit oil, hydroxyproline, isotridecyl isononanoate, cetyl ethylhexanoate, diethylhexyl succinate, squalane, stearic acid, sorbitan sesquistearate, triethylhexanoin, hydrogenated polyisobutene, hydrogenated lecithin, PG dicaprylate, isostearic acid hydrogenated castor oil, sorbitan isostearate, diisostearyl malate, BG, sodium lauroyl aspartate, DPG, isostearyl alcohol or the like is used.

As the ultraviolet protector, in addition to ethylhexyl methoxycinnamate, titanium oxide, zinc oxide or the like, which are listed as the synthetic inorganic powder, is used.

When the above materials are emulsified and used, water is used as a medium.

For example, when the kraft lignin of the invention is used for a foundation, the raw materials are mixed, then formed and used as a foundation.

The kraft lignin of the invention can be used especially as a sunscreen of the cosmetic uses. The sunscreen mainly contains the ultraviolet-cutting agent of the invention, an oil-based component and a water-based component such as water and an alcohol and is preferably a liquid, an emulsion, a gel or a cream. Moreover, a pack, a mask, a nonwoven cloth or the like containing the sunscreen can also be used.

As the oil-based component, one kind or two or more kinds of a higher alcohol, a hydrocarbon oil, an ester oil, silicone and the like can be used. Moreover, a hydrocarbon wax can also be contained. When the oil-based component is used for the sunscreen, the oil-based component content based on the total sunscreen weight is preferably 1 to 99% by weight.

As the water-based component, water which is used for a cosmetic, a quasi-drug or the like can be used, and purified water, ion-exchanged water, tap water or the like can be used. Moreover, as the alcohol, a water-soluble alcohol can be used, and one kind or two or more kinds selected from a lower alcohol, a polyhydric alcohol, a polyhydric alcohol polymer, glycerin monoalkyl ether, sugar alcohol, a monosaccharide, an oligosaccharide, a derivative thereof and the like can be used.

In addition to the components, the sunscreen can appropriately contain, according to the need, powder other than the ultraviolet-cutting agent of the invention, an ultraviolet absorber other than the ultraviolet-cutting agent of the invention, a surfactant, a water-soluble polymer, a moisturizer, a film-forming agent, a sequestering agent, an amino acid, an organic amine, a polymer emulsion, a pH-adjusting agent, a skin nutrient, a vitamin, an antioxidant, a fragrance or the like.

As the powder other than the kraft lignin of the invention, a known usual powder which is used for a general sunscreen can be used, and an inorganic powder such as magnesium oxide, calcium carbonate, talc, synthetic mica, mica, kaolin, montmorillonite, zeolite, ceramic powder, boron nitride and alumina, an inorganic pigment such as iron oxide, titanium oxide, zinc oxide and ultramarine, an organic pigment such as pigment red 202, pigment red 204, pigment yellow 4, pigment yellow 202, pigment blue 1, pigment blue 201, pigment green 3, pigment green 204, pigment orange 201 and pigment orange 207, a natural dye such as turmeric dye, carotene dye, cochineal dye, *Monascus purpureus* dye, chlorophyll dye, a *Spirulina* dye and a squid ink dye, a laked natural dye, an effect pigment such as titanium oxide-coated mica and titanium oxide-coated glass or the like can be used.

As the ultraviolet absorber other than the ultraviolet-cutting agent of the invention, a benzoic acid ultraviolet absorber, a salicylic acid ultraviolet absorber, a cinnamic acid ultraviolet absorber, a benzophenone ultraviolet absorber or the like can be used.

As the surfactant, an anionic surfactant, a cationic surfactant, an amphoteric surfactant, a lipophilic nonionic surfactant, a hydrophilic nonionic surfactant or the like can be used. Here, the surfactant content based on the total mass of the cosmetic composition of the invention is preferably 5 to 15% by mass.

As the water-soluble polymer, natural and synthetic water-soluble polymers can be used. As the natural water-soluble polymer, a plant polymer such as gum arabic, guar gum and starch, a microorganism-derived polymer or an animal polymer such as collagen and gelatin can be used. As the synthetic water-soluble polymer, s starch polymer, a cellulose polymer, an alginic acid polymer, a vinyl polymer, an acrylic polymer or the like can be used.

As the moisturizer, protein, a mucopolysaccharide, collagen, elastin or keratin can be used.

As the film-forming agent, an anionic filming agent, a cationic filming agent or a nonionic filming agent can be used.

As the sequestering agent, tetrasodium edetate, sodium citrate, sodium polyphosphate, ascorbic acid or the like can be used, and as the amino acid, a neutral amino acid, a basic amino acid, an amino acid derivative or the like can be used. As the organic amine, diethanolamine, triethanolamine, triisopropanolamine or the like can be used, and as the polymer emulsion, an acrylic resin emulsion, natural rubber latex, a silicone emulsion or the like can be used. As the pH-adjusting agent, a buffer such as sodium lactate, sodium citrate and sodium succinate or the like can be used, and as the vitamin, vitamin A, vitamin B1, vitamin B2, vitamin C, vitamin E or the like can be used. As the antioxidant, a tocopherol, dibutylhydroxytoluene, a gallic acid ester or the like can be used, and as the fragrance, a known usual fragrance can be used as long as the fragrance is generally used for a sunscreen.

Furthermore, as another component, a preservative, an antiphlogistic agent, a skin lightening agent, an activator, a blood circulation-improving agent, an anti-inflammatory agent, a bacteria-regulating agent, an antiviral agent or the like can be used.

The sunscreen can be prepared by dissolving the components which are heated to 20 to 80°C or are at normal temperature with stirring. As the stirring method, a known method can be used, and for example, an apparatus such as a blade-type stirrer, a disper and a homo-mixer can be used. By cooling the mixture which has become homogeneous by dissolving to normal temperature, the sunscreen can be obtained. Depending on the preparation of the cosmetic composition, a liquid cosmetic, such as a liquid, a paste and a gel, can be obtained during cooling.

### (Paint Use)

When the kraft lignin of the invention is used for a paint as a colorant, an ultraviolet protector or an antioxidant, the resin used as the paint is any of various resins such as an acrylic resin, a melamine resin, an epoxy resin, a polyester resin, a polyurethane resin, a polyamide resin and a phenolic resin.

Examples of the solvent used for the paint include an aromatic solvent such as toluene, xylene and methoxybenzene, an acetic acid ester solvent such as ethyl acetate, butyl acetate, propylene glycol monomethyl ether acetate and propylene glycol monoethyl ether acetate, a propionate solvent such as ethoxyethyl propionate, an alcohol solvent such as methanol, ethanol, propanol, n-butanol and isobutanol, an ether solvent such as butyl Cellosolve, propylene glycol monomethyl ether, diethylene glycol ethyl ether and diethylene glycol dimethyl ether, a ketone solvent such as methyl ethyl ketone, methyl isobutyl ketone and cyclohexanone, an aliphatic hydrocarbon solvent such as hexane, a nitrogen compound solvent such as N,N-dimethylformamide, γ-butyrolactam, n-methyl-2-pyrrolidone, aniline and pyridine, a lactone solvent such as γ-butyrolactone, a carbamic acid ester such as a mixture of methyl carbamate and ethyl carbamate at 48:52, water and the like. As the solvent, in particular, a polar solvent which is soluble in water, such as a propionate, an alcohol, an ether, a ketone, a nitrogen compound, a lactone and water, is suitable.

Moreover, when a resin composition for a paint is obtained by dispersing or mixing the kraft lignin in a liquid resin, a general additive, for example, a dispersant, a filler, a paint adjuvant, a desiccant, a plasticizer and/or an adjuvant compound, can be used. This is achieved by dispersing or mixing by collecting all the components, either each component individually or some thereof together, or by adding all the components at once.

Examples of the dispersing machine for dispersing the composition containing the kraft lignin of the invention which is prepared according to the use as described above include known dispersing machines such as a disper, a homo-mixer, a paint conditioner, a scandex, a bead mill, an attritor, a ball mill, a two-roll machine, a three-roll machine and a pressurized kneader, but the dispersing machine is not limited thereto. When the compound is dispersed, the resin and the solvent are added for dispersion in such a manner that the mixture has a viscosity which can be dispersed with such a dispersing machine. The high-concentration paint base after dispersion has a solid content of 5 to 20% and is subjected to use as a paint after further mixing the resin and the solvent therein.

### (Automotive Paint Use)

When the kraft lignin of the invention is used for an automotive paint as a colorant, the resin used as the paint is any of various resins such as an acrylic resin, a melamine resin, an epoxy resin, a polyester resin, a polyurethane resin, a polyamide resin and a phenolic resin.

The automotive paint is preferably a water-based paint or an electrically deposited powder paint in view of the recent restriction on VOCs.

When a resin composition for a paint is obtained by dispersing or mixing the kraft lignin in a liquid resin or by dry blending, a general additive, for example, a dispersant, a filler, a paint adjuvant, a desiccant, a plasticizer and/or an adjuvant compound, can be used. This is achieved by dispersing or mixing by collecting all the components, either each component individually or some thereof together, or by adding all the components at once.

### (Inkjet Ink Use)

The kraft lignin of the invention can be preferably used for an inkjet ink and can be preferably used for a water-based inkjet ink especially as a water-based compound dispersion obtained by dispersing using a dispersant or the like for the kraft lignin. The water-based compound dispersion can be prepared by producing a high-concentration aqueous dispersion of the kraft lignin of the invention (compound paste), diluting the dispersion with a water-soluble solvent and/or water and adding another additive according to the need.

The method for obtaining the compound paste by dispersing the kraft lignin of the invention in the water-soluble solvent and/or water is not particularly limited, and a known dispersing method is preferably used. At this time, as the dispersant used, a known compound dispersant may be used for dispersing in water, or a surfactant may also be used. The compound dispersant is preferably a water-based resin, and preferable examples thereof include a polyvinyl alcohol, a polyvinylpyrrolidone, a urethane resin having an anionic group or a cationic group, a radical copolymer resin having an anionic group or a cationic group and the like. Examples of the radical copolymer resin having an anionic group or a cationic group include an acrylic resin such as an acrylic acid-acrylic acid ester copolymer, a styrene-acrylic resin such as a styrene-acrylic acid copolymer, a styrene-methacrylic acid copolymer, a styrene-methacrylic acid-acrylic acid ester copolymer, a styrene-α-methylstyrene-acrylic acid copolymer and a styrene-α-methylstyrene-acrylic acid-acrylic acid ester copolymer, a styrene-maleic acid copolymer, a styrene-maleic anhydride copolymer, a vinylnaphthalene-acrylic acid copolymer and salts of the water-based resins.

Examples of the compound for forming a salt of the copolymer include an alkali metal hydroxide such as sodium hydroxide, potassium hydroxide and lithium hydroxide, diethylamine, ammonia, ethylamine, triethylamine, propylamine, isopropylamine, dipropylamine, butylamine, isobutylamine, triethanolamine, diethanolamine, aminomethyl propanol, morpholine and the like. The amount of the compound used for forming such a salt is preferably the neutralization equivalent of the copolymer or more.

Moreover, of course, a commercial product can also be used. As the commercial product, Ajisper PB series, which are products manufactured by Ajinomoto Fine-Techno Co., Inc., Disperbyk series and BYK-series of BYK-Chemie Japan, EFKA series manufactured by BASF Japan Ltd. and the like can be used.

Moreover, examples of the dispersing method include (1) and (2) below.
(1) A method of adding the kraft lignin to a water-based medium containing a kraft lignin dispersant and water, then dispersing the kraft lignin in the water-based medium using a stirring·dispersing machine and thus preparing a kraft lignin paste.
(2) A method of kneading the kraft lignin and a kraft lignin dispersant using a kneader such as a two-roll kneader and a mixer, adding the obtained kneaded material to a water-based medium containing water and preparing a compound paste using a stirring·dispersing machine.

### (Toner Use)

The kraft lignin of the invention can also be used for a toner coloring use.

When a toner for electrostatic image development is obtained, a thermoplastic resin which is solid at normal temperature and which has film-forming property, such as a polyester resin, a polyamide resin, a styrene resin and an acrylic resin, is used as a resin for dispersing.

The toner for electrostatic image development which is produced using the kraft lignin of the invention as a component can be used as a one-component color magnetic toner containing a magnetic material in the toner (a color toner for magnetic one-component development), a nonmagnetic one-component color toner containing no magnetic material (a color toner for nonmagnetic one-component development) or a color toner for a two-component color development agent in which carrier has been mixed (a color toner for two-component development).

The one-component color magnetic toner can be composed of, for example, a colorant, a binder resin, a magnetic powder, another additive such as a charge control agent (CCA) and a mold release agent and the like, like those generally used.

Although the used amount of the kraft lignin of the invention in the toner for electrostatic image development is not particularly limited, the kraft lignin is preferably used at a ratio of 0.5 to 25 parts by mass based on 100 parts by mass of the binder resin, and the amount is further preferably four to 10 parts by mass based on 100 parts by mass of the binder resin to make the electrostatic property of the colorant itself to become further remarkable.

Any of the known usual resins which are listed as examples of the thermoplastic resin can be used as the binder resin used for the toner for electrostatic image development, but any of a synthetic resin, a natural resin, natural rubber, synthetic rubber, a synthetic wax and the like which are adhesive when heat or pressure is applied can be used.

### (Black Matrix·Photo Spacer Uses)

The kraft lignin of the invention can be used for forming a pattern of the black matrix part in a color filter or for a photo spacer for LCD by a known method. Typically, a photosensitive composition for pattern formation containing the kraft lignin of the invention and a photosensitive resin as essential components can be obtained.

To prepare the photosensitive composition for pattern formation, for example, the kraft lignin of the invention, a photosensitive resin, a photopolymerization initiator and an organic solvent for dissolving the resin are mixed as essential components. A general production method thereof is a preparation method of preparing a dispersion using the kraft lignin of the invention and an organic solvent and using a dispersant according to the need and then adding a photosensitive resin or the like thereto.

For the kraft lignin of the invention used for the photosensitive composition for pattern formation, a yellow compound can be used according to the need. Examples of the dispersant which is used according to the need include DISPERBYK (registered trademark)-130, -161, -162, -163, -170, -LPN-6919 and -LPN-21116 of BYK-Chemie GmbH and the like. Moreover, a leveling agent, a coupling agent, a cationic surfactant or the like can also be used in combination.

Examples of the organic solvent include an aromatic solvent such as toluene, xylene and methoxybenzene, an acetic acid ester solvent such as ethyl acetate, butyl acetate, propylene glycol monomethyl ether acetate and propylene glycol monoethyl ether acetate, a propionate solvent such as ethoxyethyl propionate, an alcohol solvent such as methanol and ethanol, an ether solvent such as butyl Cellosolve, propylene glycol monomethyl ether, diethylene glycol ethyl ether and diethylene glycol dimethyl ether, a ketone solvent such as methyl ethyl ketone, methyl isobutyl ketone and cyclohexanone, an aliphatic hydrocarbon solvent such as hexane, a nitrogen compound solvent such as N,N-dimethylformamide, γ-butyrolactam, n-methyl-2-pyrrolidone, aniline and pyridine, a lactone solvent such as γ-butyrolactone, a carbamic acid ester such as a mixture of methyl carbamate and ethyl carbamate at 48:52, water and the like. As the organic solvent, in particular, a polar solvent which is soluble in water, such as a propionate, an alcohol, an ether, a ketone, a nitrogen compound, a lactone and water is suitable.

Examples of the photosensitive resin which can be used include thermoplastic resins such as an urethane resin, an acrylic resin, a polyamidic acid resin, a polyimide resin, a styrene maleic acid resin and a styrene maleic anhydride resin and photopolymerizable monomers including a bifunctional monomer such as 1,6-hexanediol diacrylate, ethylene glycol diacrylate, neopentyl glycol diacrylate, triethylene glycol diacrylate, bis(acryloxyethoxy)bisphenol A and 3-methylpentanediol diacrylate, a polyfunctional monomer such as trimethylolpropane triacrylate, pentaerythritol triacrylate, tris(2-hydroxyethyl)isocyanurate, dipentaerythritol hexaacrylate and dipentaerythritol pentaacrylate and the like.

Examples of the photopolymerization initiator include acetophenone, benzophenone, benzyldimethylketal, benzoyl peroxide, 2-chlorthioxanthone, 1,3-bis(4'-azidobenzal)-2-propane, 1,3-bis(4'-azidobenzal)-2-propane-2'-sulfonic acid, 4,4'-diazidostilbene-2,2'-disulfonate and the like.

The photosensitive composition for a black matrix thus prepared can be formed into a black matrix or a spacer by conducting pattern exposure with ultraviolet rays through a photo mask and then washing off the unexposed part with an organic solvent, alkaline water or the like.

### EXAMPLES

The invention is described in further detail below referring to Examples, but the invention is not limited to the Examples. Moreover, "%" in the compositions of the Examples below mean "% by weight".

### (Measurement Method of Particle Size Distribution)

A particle size distribution was measured with Microtrac Nanowave II manufactured by MicrotracBEL Corp. The particle size distribution was measured using water as the measurement medium by frequency analysis of the dynamic light scattering of a semiconductor laser having a wavelength of 780 nm.

### (Color Measurement)

An integrating sphere unit ISN-923 was attached to a spectrophotometer V-770 manufactured by JASCO Corporation, and 0.1 g of a measurement sample was put into a powder cell PSH-002. From the measured reflectance spectrum of 360 to 830 nm, color measurement was conducted by the L*a*b* color system in accordance with JIS Z 8781-4: 2013, CIE No.15:2004, ISO11664-4: 2008, ASTM E308:2008.

### (Measurement Method of SPF)

The SPF measurement was made using an UV-2000S SPF analyzer manufactured by Labsphere based on ISO24443. A sample was applied to HERIOPLATE HD6 at a ratio of 1.0 mg/cm², and thus an SPF measurement sample was produced. The transmittance was measured at nine different measurement points per one piece of SPF measurement sample, and the SPF was calculated from the transmittance values.

### (Measurement of Hydroxy Group Amount)

The amount of hydroxy groups, which are involved in the antioxidative ability of the kraft lignin, was measured with a Fourier transform infrared spectrophotometer FT/IR-6100 manufactured by JASCO Corporation as the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹). An ATR adopter was attached for the measurement, and the kraft lignin powder was directly measured. The hydroxy group amount was determined as the ratio of the hydroxy groups and the hydrocarbon groups. The ratio of the hydroxy groups and the hydrocarbon groups was determined from the ratio of the heights of the peak of 3000 to 3600 cm⁻¹ showing the hydroxy groups and the peak of 1480 to 1540 cm⁻¹ showing the hydrocarbon groups.

### (Measurement of Active Radical-Scavenging Ability)

The active radical-scavenging ability, which is involved in the antioxidative ability of the kraft lignin, was measured using 2,2-diphenyl-1-picrylhydrazyl (DPPH). The measurement method and the measurement results are shown below.

Isopropanol was added to 20 mg of a kraft lignin, and thus a crude 20 mg/L isopropanol (IPA) dispersion was obtained. The crude dispersion was ultrasonically dispersed using UP400S manufactured by Hielscher for 30 seconds, and a kraft lignin/IPA dispersion was thus obtained. A 2,2-diphenyl-1-picrylhydrazyl (DPPH) 0.20 mM IPA solution was separately prepared and mixed with the kraft lignin/IPA dispersion in the same amounts, and the mixture was left still for two hours at room temperature. The absorbance at 518 nm of the solution after leaving still was measured, and the remaining DPPH amount was determined from a calibration curve determined from the absorbances at 518 nm of 0.20 mM, 0.10 mM, 0.05 mM and 0.02 mM DPPH/IPA solutions. The remaining DPPH amount was subtracted from 0.1 mM, which is the initial DPPH concentration, and thus the DPPH active radical-scavenging ability of the kraft lignin was determined. It was decided that the DPPH radical-scavenging ability was indicated with unit DPPH/mg as the DPPH active radical-scavenging ability per 1 mg of the kraft lignin.

### (Example 1)

Ten parts by weight of a kraft lignin (manufactured by Nippon Paper Industries Co., Ltd.) were dissolved in 40 parts by weight of ethanol (JIS 1st Grade manufactured by FUJIFILM Wako Pure Chemical Corporation), and the insoluble matter was removed by filtration with filter paper. While stirring the obtained filtrate, 200 parts by weight of ice were added, and thus the kraft lignin was deposited. The obtained kraft lignin suspension was filtered, and the filtration residue was washed with 200 parts by weight of ion-exchanged water. The filtration and the washing with ion-exchanged water were repeated twice, and the wet cake of the kraft lignin was vacuum-dried at 40°C for two hours. The obtained dried material was pulverized with a mortar, and three parts by weight of a kraft lignin were obtained.

The median diameter, the color, the SPF value, the PA value, the ratio of hydroxy groups and hydrocarbon groups and the active radical-scavenging ability of the kraft lignin were measured. As a result, the median diameter was 0.581 µm, the color was L* = 73.39, a* = 3.14 and b* = 20.69, the SPF value was 21.6, the PA value was 20.8, the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹) was 3.30, and the active radical-scavenging ability was 1.57 mg DPPH/mg.

### (Example 2)

Four parts by weight of a kraft lignin were obtained by the same method as the method in Example 1 except that ethanol was replaced with methanol (JIS 1st Grade manufactured by FUJIFILM Wako Pure Chemical Corporation).

The median diameter, the color, the SPF value, the PA value, the ratio of hydroxy groups and hydrocarbon groups and the active radical-scavenging ability of the kraft lignin were measured by the same methods as those in Example 1. As a result, the median diameter was 0.672 µm, the hue was L* = 70.71, a* = 3.55 and b* = 20.53, the SPF value was 17.5, the PA value was 17.0, the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹) was 3.62, and the active radical-scavenging ability was 1.27 mg DPPH/mg.

### (Example 3)

Eight parts by weight of a kraft lignin were obtained by the same method as the method in Example 1 except that ethanol was replaced with ethylene glycol.

The median diameter, the color, the SPF value, the PA value, the ratio of hydroxy groups and hydrocarbon groups and the active radical-scavenging ability of the kraft lignin were measured by the same methods as those in Example 1. As a result, the median diameter was 0.629 µm, the color was L* = 61.05, a* = 4.83 and b* = 17.06, the SPF value was 41.1, the PA value was 38.9, the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹) was 4.07, and the active radical-scavenging ability was 1.06 mg DPPH/mg.

### (Example 4)

Seven parts by weight of a kraft lignin were obtained by the same method as the method in Example 1 except that ethanol was replaced with diethylene glycol.

The median diameter, the color, the SPF value, the PA value, the ratio of hydroxy groups and hydrocarbon groups and the active radical-scavenging ability of the kraft lignin were measured by the same methods as those in Example 1. As a result, the median diameter was 0.308 µm, the color was L* = 63.93, a* = 4.26 and b* = 17.37, the SPF value was 22.2, the PA value was 21.1, the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹) was 5.00, and the active radical-scavenging ability was 1.04 mg DPPH/mg.

### (Example 5)

Eight parts by weight of a kraft lignin were obtained by the same method as the method in Example 1 except that ethanol was replaced with dimethylformamide.

The median diameter, the color, the SPF value, the PA value, the ratio of hydroxy groups and hydrocarbon groups and the active radical-scavenging ability of the kraft lignin were measured by the same methods as those in Example 1. As a result, the median diameter was 0.648 µm, the color was L* = 67.98, a* = 3.86 and b* = 18.29, the SPF value was 11.6, the PA value was 11.4, the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹) was 4.61, and the active radical-scavenging ability was 0.96 mg DPPH/mg.

### (Example 6)

Eight parts by weight of a kraft lignin were obtained by the same method as the method in Example 1 except that ethanol was replaced with N,N-dimethylpyrrolidinone.

The median diameter, the color, the SPF value, the PA value, the ratio of hydroxy groups and hydrocarbon groups and the active radical-scavenging ability of the kraft lignin were measured by the same methods as those in Example 1. As a result, the median diameter was 0.505 µm, the color was L* = 68.69, a* = 3.61 and b* = 18.21, the SPF value was 15.1, the PA value was 14.8, the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹) was 4.64, and the active radical-scavenging ability was 1.03 mg DPPH/mg.

### (Example 7)

Ten parts by weight of a kraft lignin were dissolved in 40 parts by weight of ethanol, and the insoluble matter was removed by filtration with filter paper. The obtained filtrate was added to 200 parts by weight of ice, and thus the kraft lignin was deposited. The kraft lignin suspension was filtered, and the filtration residue was washed with 200 parts by weight of ion-exchanged water. The filtration and the washing with ion-exchanged water were repeated twice, and the wet cake of the kraft lignin was vacuum-dried at 40°C for two hours. The obtained dried material was pulverized with a mortar, and three parts by weight of a kraft lignin were obtained.

The median diameter, the color, the SPF value, the PA value, the ratio of hydroxy groups and hydrocarbon groups and the active radical-scavenging ability of the kraft lignin were measured by the same methods as those in Example 1. As a result, the median diameter was 0.595 µm, the color was L* = 72.34, a* = 3.28 and b* = 20.64, the SPF value was 20.6, the PA value was 19.8, the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹) was 3.2, and the active radical-scavenging ability was 1.50 mg DPPH/mg.

### (Example 8)

Ten parts by weight of a kraft lignin were dissolved in 40 parts by weight of an industrial alcohol (Solmix AP-7), and the insoluble matter was removed by filtration. Twenty parts by weight of ethylene glycol were added to the obtained filtrate, and distillation at reduced pressure was conducted at 80°C. Thus, 25 parts by weight of an ethylene glycol solution of the kraft lignin were obtained. Twenty-five parts by weight of 10% sodium chloride solution were added to the obtained ethylene glycol solution of the kraft lignin, and the kraft lignin was deposited. The kraft lignin suspension was filtered, and the filtration residue was washed with 45 parts by weight of ion-exchanged water. The filtration and the washing with ion-exchanged water were repeated five times, and the wet cake of the kraft lignin was dried at 40°C for two hours. The obtained dried material was pulverized with a mixer, and five parts by weight of a kraft lignin were obtained.

The median diameter, the color, the SPF value, the PA value, the ratio of hydroxy groups and hydrocarbon groups and the active radical-scavenging ability of the kraft lignin were measured by the same methods as those in Example 1. As a result, the median diameter was 0.595 µm, the color was L* = 69.72, a* = 3.56 and b* = 17.73, the SPF value was 23.6, the PA value was 21.8, the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹) was 3.4, and the active radical-scavenging ability was 1.24 mg DPPH/mg.

### (Comparative Example 1)

The median diameter, the color, the SPF value, the PA value, the ratio of hydroxy groups and hydrocarbon groups and the active radical-scavenging ability of the kraft lignin (manufactured by Nippon Paper Industries Co., Ltd.) before the treatment were measured in the same manner. The median diameter was 0.823 µm, the color was L* = 51.19, a* = 6.58 and b* = 16.41, the SPF value was 7.9, the PA value was 2.3, the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹) was 2.83, and the active radical-scavenging ability was 0.28 mg DPPH/mg.

### (Comparative Example 2)

It was attempted to prepare fine particles by the same method as the method in Example 1 except that ice was replaced with water, but the kraft lignin precipitated into a mass, and particles could not be prepared.

### (Comparative Example 3)

It was attempted to prepare particles by the same method as the method in Example 1 except that ethanol was replaced with isopropanol, but the kraft lignin did not dissolve, and fine particles could not be prepared.

### (Comparative Example 4)

It was attempted to prepare particles by the same method as the method in Example 1 except that ethanol was replaced with normal butanol, but the kraft lignin did not dissolve, and fine particles could not be prepared.

### (Comparative Example 5)

Fine particles were prepared by the same method as the method in Example 1 except that ethanol was replaced with acetone, and six parts by weight of kraft lignin fine particles were obtained.

The median diameter, the color, the SPF value, the PA value, the ratio of hydroxy groups and hydrocarbon groups and the active radical-scavenging ability of the kraft lignin particles were measured by the same methods as those in Example 1. As a result, the median diameter was 2.11 µm, the color was L* = 54.25, a* = 6.05 and b* = 17.39, the SPF value was 25.5, the PA value was 23.3, the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹) was 3.66, and the active radical-scavenging ability was 0.77 mg DPPH/mg.

### (Comparative Example 6)

It was attempted to prepare fine particles by the same method as the method in Example 1 except that ethanol was replaced with methyl ethyl ketone, but the kraft lignin precipitated into a mass, and particles could not be prepared.

### (Comparative Example 7)

It was attempted to prepare fine particles by the same method as the method in Example 1 except that ethanol was replaced with methyl isobutyl ketone, but the kraft lignin did not dissolve, and fine particles could not be prepared.

### (Comparative Example 8)

It was attempted to prepare fine particles by the same method as the method in Example 1 except that ethanol was replaced with butyl Cellosolve, but the kraft lignin precipitated into a mass, and fine particles could not be prepared.

### (Comparative Example 9)

Nine parts by weight of kraft lignin fine particles were obtained by the same method as the method in Example 1 except that ethanol was replaced with butyl Carbitol.

The median diameter, the color, the SPF value, the PA value, the ratio of hydroxy groups and hydrocarbon groups and the active radical-scavenging ability of the kraft lignin fine particles were measured by the same methods as those in Example 1. As a result, the median diameter was 5.78 µm, the color was L* = 60.00, a* = 6.84 and b* = 23.69, the SPF value was 20.7, the PA value was 20.6, the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹) was 4.94, and the active radical-scavenging ability was 0.61 mg DPPH/mg.

### (Comparative Example 10)

Eight parts by weight of kraft lignin fine particles were obtained by the same method as the method in Example 1 except that ethanol was replaced with tetrahydrofuran.

The median diameter, the color, the SPF value, the PA value, the ratio of hydroxy groups and hydrocarbon groups and the active radical-scavenging ability of the kraft lignin fine particles were measured by the same methods as those in Example 1. As a result, the median diameter was 1.92 µm, the color was L* = 54.24, a* = 6.67 and b* = 18.57, the SPF value was 29.7, the PA value was 17.6, the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹) was 3.64, and the active radical-scavenging ability was 0.14 mg DPPH/mg.

### (Comparative Example 11)

It was attempted to prepare fine particles by the same method as the method in Example 1 except that ethanol was replaced with hexyl Carbitol, but the kraft lignin did not dissolve, and fine particles could not be prepared.

### (Comparative Example 12)

Eight parts by weight of kraft lignin fine particles were obtained by the same method as that in Example 1 except that 10 parts by weight of the kraft lignin were dissolved in 40 parts by weight of N,N-dimethylpyrrolidinone and that the solution was then heated at 180°C for two hours.

The median diameter, the color, the SPF value, the PA value, the ratio of hydroxy groups and hydrocarbon groups and the active radical-scavenging ability of the kraft lignin fine particles were measured by the same methods as those in Example 1. As a result, the median diameter was 0.415 µm, the color was L* = 59.57, a* = 4.64 and b* = 16.15, the SPF value was 29.7, the PA value was 18.0, the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹) was 2.87, and the active radical-scavenging ability was 0.904 mg DPPH/mg.

### (Evaluation Example 1)

A foundation cosmetic using the kraft lignin of the invention was evaluated.

The foundation cosmetics for evaluation were blended with the composition shown in Table 1. Comparative evaluation of the kraft lignin described in Example 8 of the invention, the kraft lignin before the treatment described in Comparative Example 1, four commercial lignin materials and a commercial foundation cosmetic was conducted. The evaluation was conducted by SPF measurement.

The composition of the foundations for evaluation is shown in Table 1.

**[Table 1]**

| | Foundation Composition for Evaluation |
|---|---|
| Mica | 29.06 |
| (Mearlmica^{®} FF) | |
| Kaolin | 5.51 |
| Bismuth oxychloride | 5.51 |
| (Pearl-Glo^{®} SF PG 1099) | |
| Zinc stearate | 1.00 |
| | |
| Zinc oxide | 4.50 |
| Red iron oxide | 0.95 |
| Yellow iron oxide | 1.00 |
| Lignin material | 50.00 |
| | |
| Glyceryl tri(caprylate/caprate) | 1.10 |
| (Myritol^{®} 312) | |
| Cocoglyceryl | 0.60 |
| (Myritol^{®} 331) | |
| Cocoalkyl (caprylate/caprate) | 0.70 |
| (Cetiol^{®} C 5) | |
| Pentaerythrityl tetradibutyl hydroxyhydrocinnamate (Tinogard^{®} TT) | 0.06 |
| | |
| Total | 100.00 |

The SPF measurement was made using an UV-2000S SPF analyzer manufactured by Labsphere based on ISO24443. A sample was applied to HERIOPLATE HD6 at a ratio of 0.5 mg/cm², and thus an SPF measurement sample was produced. The transmittance was measured at nine different measurement points per one piece of SPF measurement sample, and the SPF was calculated from the transmittance values. The evaluation results are shown in Table 2.

**[Table 2]**

| | Commercial foundation | Example 8 | Comparative Example 1 | Lignin material A | Lignin material B | Lignin material C | Lignin material D |
|---|---|---|---|---|---|---|---|
| SPF | 4.54 | 8.57 | 3.11 | 3.16 | 3.07 | 2.95 | 3.1 |
| PA | 3.39 | 7.68 | 2.90 | 2.93 | 2.83 | 2.73 | 2.86 |

### (Evaluation Example 2)

A sunscreen cream using the kraft lignin of the invention was evaluated.

The sunscreen creams for evaluation were blended with the composition shown in Table 3. Comparative evaluation of the kraft lignin described in Example 8 of the invention, the kraft lignin before the treatment described in Comparative Example 1 and four commercial lignin materials was conducted. The evaluation was conducted by SPF measurement.

The composition of the sunscreen creams for evaluation is shown in Table 3.

**[Table 3]**

| | Control | Composition for Evaluation |
|---|---|---|
| Lignin material | | 0.050 |
| | | |
| Lanolin | 0.250 | 0.250 |
| Vaseline | 0.125 | 0.125 |
| Stearic acid | 0.200 | 0.200 |
| Liquid paraffin | 1.000 | 1.000 |
| Squalane | 1.000 | 1.000 |
| | | |
| Component B | | |
| Disodium edetate | 0.003 | 0.003 |
| Propylene glycol | 0.250 | 0.250 |
| Triethanolamine | | |
| Purified water | 1.263 | 1.263 |
| | | |
| Component C | | |
| Zinc oxide | 0.400 | 0.400 |
| Titanium oxide | | |
| Red iron oxide | 0.005 | 0.005 |
| Yellow iron oxide | | |
| | | |
| Purified water | 0.505 | 0.455 |
| | | |
| Total | 5.000 | 5.000 |

The SPF measurement was made using an UV-2000S SPF analyzer manufactured by Labsphere based on ISO24443. A sample was applied to HERIOPLATE HD6 at a ratio of 2.0 mg/cm², and thus an SPF measurement sample was produced. The transmittance was measured at nine different measurement points per one piece of SPF measurement sample, and the SPF was calculated from the transmittance values. The evaluation results are shown in Table 4.

**[Table 4]**

| | Blank | Example 8 | Comparative Example 1 | Lignin material A | Lignin material B | Lignin material C | Lignin material D |
|---|---|---|---|---|---|---|---|
| Lignin content | - | 1% | 1% | 1% | 1% | 1% | 1% |
| Zinc oxide content | 8% | 8% | 8% | 8% | 8% | 8% | 8% |
| SPF | 20.03 | 25.94 | 15.22 | 18.33 | 12.86 | 14.06 | 13.39 |
| PA | 12.79 | 16.58 | 11.23 | 12.33 | 9.11 | 9.91 | 9.50 |

## Claims

1. A kraft lignin, wherein the ratio of the heights of the absorption peak of 1480 to 1540 cm⁻¹ and the absorption peak of 3000 to 3600 cm⁻¹ (1480 to 1540 cm⁻¹/3000 to 3600 cm⁻¹) is in the range of 3.0 to 6.0 in an infrared absorption spectrum measured with a Fourier transform infrared spectrophotometer having an ATR unit (ATR-FT-IR), and the L* value is in the range of 61 to 80 in color measurement with a spectrophotometer.

2. The kraft lignin according to claim 1 which has a median diameter (D50) of 0.1 to 0.8 µm.

3. The kraft lignin according to claim 1 or 2, wherein the SPF (Sun Protection Factor) measured using an SPF (Sun Protection Factor) analyzer is in the range of 10 to 50 when the coating amount of the kraft lignin per unit area is 1 mg/cm² in ISO 24443.

4. The kraft lignin according to claim 1 or 2, wherein the PA (Protection Grade of UVA) value measured using an SPF (Sun Protection Factor) analyzer is in the range of 10 to 50 when the coating amount of the kraft lignin per unit area is 1 mg/cm² in ISO 24443.

5. The kraft lignin according to claim 1 or 2, wherein the 2,2-diphenyl-1-picrylhydrazyl (DPPH) active radical-scavenging ability of the kraft lignin per unit weight is 1.0 to 3.0 mg DPPH/mg.

6. A method for producing the kraft lignin according to claim 1 or 2, comprising
a step of dissolving a kraft lignin in an organic solvent and thus obtaining a kraft lignin solution,
a subsequent step of adding ice or ice water to the kraft lignin solution or adding the kraft lignin solution to ice or ice water to deposit the kraft lignin and obtaining the deposited kraft lignin, and
a subsequent step of filtering, washing and drying the deposited kraft lignin and thus obtaining the kraft lignin.

7. A method for producing the kraft lignin according to claim 1 or 2, comprising
a step of dissolving a kraft lignin in an organic solvent and thus obtaining a kraft lignin solution,
a subsequent step of adding an aqueous water-soluble metal salt solution to the kraft lignin solution or adding the kraft lignin solution to an aqueous water-soluble metal salt solution to deposit the kraft lignin, and
a subsequent step of filtering, washing and drying the deposited kraft lignin and thus obtaining the kraft lignin.

8. A foundation, a sunscreen, a cosmetic, an ink, a printed matter, a paint, an automotive paint, a coating, a plastic, a fiber, a film, a black matrix resist, a photo spacer resist and a cured material thereof comprising the kraft lignin according to claim 1 or 2.
